# EUROPÄISCHE PATENTSCHRIFT

(11) **EP 0 931 058 B1**
(45) Veröffentlichungstag und Bekanntmachung des Hinweises auf die Patenterteilung: **03.05.2000**
(21) Anmeldenummer: 97943816.5
(22) Anmeldetag: 21.08.1997
(51) Int. Cl.: C07C 253/30, C07C 255/25, B01J 23/74, B01J 25/00, B01J 23/76

(54) **FÜR DIE HERSTELLUNG VON ALIPHATISCHEN ALPHA, OMEGA-AMINONITRILEN DURCH PARTIELLE HYDRIERUNG VON ALIPHATISCHEN DINITRILEN GEEIGNETE KATALYSATOREN**
CATALYSTS SUITABLE FOR PREPARING ALIPHATIC ALPHA-, OMEGA-AMINONITRILES BY PARTIAL HYDROGENATION OF ALIPHATIC DINITRILES
CATALYSEURS CONVENANT A LA PREPARATION D'ALPHA, OMEGA-AMINONITRILES ALIPHATIQUES PAR HYDROGENATION PARTIELLE DE DINITRILES ALIPHATIQUES

(30) Priorität: 10.09.1996 DE 19636768
(43) Veröffentlichungstag der Anmeldung: 28.07.1999
(73) Patentinhaber: BASF AKTIENGESELLSCHAFT, 67056 Ludwigshafen (DE)
(72) Erfinder: FLICK, Klemens, D-76863 Herxheim (DE); FISCHER, Rolf, D-69121 Heidelberg (DE); EBEL, Klaus, D-68623 Lampertheim (DE); SCHNURR, Werner, D-67273 Herxheim (DE); VOIT, Guido, D-69198 Schriesheim (DE)
(86) Internationale Anmeldenummer: EP9704547
(87) Internationale Veröffentlichungsnummer: WO9811058

(56) Entgegenhaltungen:
- WO-A-92/21650
- WO-A-93/12073
- WO-A-96/18603
- DE-A- 4 446 893
- DE-A- 4 446 895

## Beschreibung

Die vorliegende Erfindung betrifft für die Herstellung von aliphatischen alpha, omega-Aminonitrilen durch partielle Hydrierung von aliphatischen Dinitrilen geeignete Katalysatoren.

Ferner betrifft sie Verfahren zur Herstellung von aliphatischen alpha, omega-Aminonitrilen durch partielle Hydrierung von aliphatischen Dinitrilen in Gegenwart solcher Katalysatoren sowie die Verwendung der Katalysatoren für die Herstellung von aliphatischen alpha, omega-Aminonitrilen durch partielle Hydrierung von aliphatischen Dinitrilen.

Die WO 92/21650 beschreibt die Partielle Hydrierung von Adipodinitril zu 6-Aminocapronitril in Gegenwart eines Raney-Nickel-Katalysators und Ammoniak als Lösungsmittel mit einer Ausbeute von 60% bei einem Umsatz von 70%. Als Nebenprodukt entsteht 9% Hexamethylendiamin. Nachteilig an diesem Verfahren ist die geringe Standzeit des Katalysators.

In der US 2,257,814 und in der US 2,208,598 werden ebenfalls Herstellverfahren von 6-Aminocapronitril ausgehend von Adipodinitril beschrieben, wobei als Katalysatoren Raney-Cobalt, Eisen-, Nickel- und Cobalt- Katalysatoren auf verschiedenen Trägern eingesetzt werden. Nachteilig an diesen Verfahren sind die mit 50 bis 60% für technische Anwendungen zu niedrigen Selektivitäten.

Nach dem Verfahren der WO 93/16034 kann man die Ausbeute an Aminocapronitril dadurch steigern, daß man Adiponitril in Gegenwart von Raney-Nickel, einer Base wie Natrium-, Kalium-, Lithium- oder Ammoniumhydroxid und einer Übergangsmetall-Komplexverbindung, mit beispielsweise Eisen, Cobalt, Chrom oder Wolfram als Übergangsmetalle, und eines Lösungsmittels hydriert. Nach diesem Verfahren werden bei Umsetzen im Bereich von 45 bis 60% quantitative Ausbeuten an Aminocapronitril beschrieben. Nachteilig an diesem Verfahren ist die Aufarbeitung der zumeist toxischen Übergangsmetall-Komplexverbindungen aus den erhaltenen Reaktionsgemischen.

In der EP-A 161,419 wird die partielle Hydrierung von Adipodinitril unter Verwendung eines Rhodium-haltigen Katalysators auf einem Magnesiumoxid-Träger beschrieben. Bei einem Umsatz von 70% wird eine Selektivität von 94% erreicht. Nachteilig ist die aufwendige Herstellmethode der Rh/MgO-Katalysatoren (s. J. of Cat. 112 (1988), S. 145-156).

Die DE-A 4,235,466 beschreibt die Festbetthydrierung von Adiponitril zu 6-Aminocapronitril an nach einer speziellen Methode aus Eisenerz hergestellten Eisenschwamm-Katalysatoren (Vollkontakt), die nachträglich mit Cobalt-, Titan-, Mangan-, Chrom-, Molybdän-, Ruthenium- oder Iridium dotiert wurden. Aufgrund der geringen Oberfläche (0,8 m²/g) zeigen diese Katalysatoren in der Regel erst bei hohen Drücken und hohen Temperaturen eine brauchbare Aktivität. Ein weiterer Nachteil dieses Verfahrens ist der rasche Aktivitätsverlust: trotz Reduktion der Adiponitril- und Wasserstoff-Belastung, was üblicherweise zu einer Umsatzerhöhung führt, ging gemäß Beispiel 7 der Umsatz innerhalb von 24 h um 5 % zurück.

Die DE-A 848,654 beschreibt die kontinuierliche Festbetthydrierung von Adipodinitril an Palladium auf Kieselgel sowie an Metallen der achten Gruppe des Periodensystems, wobei diese Metalle bevorzugt als Spinelle eingesetzt werden. Wesentlicher Nachteil dieser Katalysatoren ist deren unbefriedigende Standzeit.

Aufgabe der vorliegenden Erfindung war es, für die Herstellung von aliphatischen alpha, omega-Aminonitrilen durch partielle Hydrierung von aliphatischen Dinitrilen geeignete Katalysatoren bereitzustellen, die die genannten Nachteile nicht aufweisen und eine hohe Selektivität hinsichtlich der alpha, omega-Aminonitrile und hinsichtlich der Summe aus alpha, omega-Aminonitrile und alpha, omega-Diamine aufweisen.

Demgemäß wurden für die Herstellung von aliphatischen alpha, omega-Aminonitrilen durch partielle Hydrierung von aliphatischen Dinitrilen geeignete Katalysatoren, enthaltend
(a) metallisches Cobalt, eine Cobalt-Verbindung oder deren Gemische, wobei der Anteil an metallischem Cobalt bezogen auf (a) 20 bis 100 Gew. -% beträgt,
(b) 10 bis 70 Gew.-% bezogen auf (a) metallisches Eisen, Eisenoxid, eine weitere Eisenverbindung oder deren Gemische, wobei der Anteil an Eisenoxid bezogen auf (b) 20 bis 100 Gew. -% beträgt,
(c) 0 bis 1 Gew. -% bezogen auf die Summe aus (a) und (b) eine Verbindung auf der Basis eines Alkalimetalls, Erdalkalimetalls oder Zink
gefunden.

Ferner wurden Verfahren zur Herstellung von aliphatischen alpha, omega-Aminonitrilen durch partielle Hydrierung von aliphatischen Dinitrilen in Gegenwart solcher Katalysatoren sowie die Verwendung der Katalysatoren für die Herstellung von aliphatischen alpha, omega-Aminonitrilen durch partielle Hydrierung von aliphatischen Dinitrilen gefunden.

Bevorzugt sind solche Katalysatoren, deren Anteil in dem Katalysator-Vorläufer vor der Aktivierung mit Wasserstoff oder einer Gasmischung, die Wasserstoff und ein Inertgas wie Stickstoff enthält, an einer oder mehrerer Co-Verbindungen, berechnet als Cobalt-II-oxid, 10 bis 80 Gew.-%, vorzugsweise 20 bis 70 Gew.-%, insbesondere 30 bis 60 Gew.-% beträgt.

Bevorzugt sind solche Katalysatoren, deren Anteil in dem Katalysator-Vorläufer vor der Aktivierung mit Wasserstoff oder einer Gasmischung, die Wasserstoff und ein Inertgas wie Stickstoff enthält, an einer oder mehrerer Fe-Verbindungen, berechnet als Eisen-III-oxid, 20 bis 90 Gew.-%, vorzugsweise 30 bis 80 Gew.-%, insbesondere 40 bis 70 Gew.-% beträgt.

Bei den erfindungsgemäß einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man einen oder mehrere Vorläufer der Komponente (a) zusammen mit Vorläufer der Komponente (b) und gewünschtenfalls mit einem oder mehrere Vorläufer der Spurenkomponente (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), (b) und gewünschtenfalls (c) auf den Träger nach an sich bekannten Methoden aufsprüht.

Als Vorläufer der Komponenten (a) und (b) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponente (c) kommen in der Regel gut wasserlösliche Salze der Alkalimetalle oder Erdalkalimetalle, wie Lithium, Natrium, Kalium, Rubidium, Cäsium, Magnesium oder Calcium, oder Zink sowie deren Gemische, wie Hydroxide, Carbonate, Nitrate, Chloride, Acetate, Formiate und Sulfate in Betracht, vorzugsweise Carbonate und Hydroxide.

Die Fällung erfolgt im allgemeinen aus wäßrigen Lösungen, wahlweise durch Zugabe von Fällungsreagenzien, durch Änderung des pH-Wertes oder durch Änderung der Temperatur.

Als Fällungsreagenzien können beispielsweise Ammoniumcarbonat oder Hydroxide oder Karbonate der Alkalimetalle eingesetzt werden. Werden Alkalimetall-Verbindungen Reagenzien eingesetzt, so empfiehlt es sich, die Niederschläge beispielsweise durch Auswaschen mit Wasser von anhaftenden Alkalimetall-Verbindungen zu befreien. Dies kann direkt nach der Abtrennung des Niederschlags von der Mutterlauge oder nach einem Trocknungs- und Calzinierschritt durchgeführt werden. Die Trocknung kann in an sich bekannter Weise, vorzugsweise in Sprühtürmen durchgeführt, wobei man den Niederschlag in der Regel in einer Flüssigkeit, vorteilhaft Wasser, aufschlämmt. Üblicherweise trocknet man die so erhaltene Katalysatormasse im allgemeinen bei Temperaturen im Bereich von 80 bis 150 °C, vorzugsweise von 80 bis 120°C vor.

Das Calcinieren nimmt man üblicherweise bei Temperaturen im Bereich von 150 bis 500°C, wobei in Einzelfällen auch Temperaturen von bis zu 1 000°C in Betracht kommen können, vorzugsweise 200 bis 450°C in einem Gasstrom aus Luft oder Stickstoff in hierfür geeigneten Apparaturen wie Horden- oder Drehrohröfen vor.

Das Pulver kann, insbesondere für den Fall, daß die Katalysatormasse in einem Festbett verwendet werden soll, zu Formkörpern, wie Strängen oder Tabletten in an sich bekannter Weise verarbeitet werden.

Bei der Herstellung von Strängen können Hilfsmittel wie anorganische Säuren, organische Säuren oder Basen wie Ammoniak zugegeben werden, wobei die Hilfsmittel Cobalt oder Eisenverbindungen enthalten können. Nach dem Verstrangen kann man die Stränge bei Temperaturen unter 200°C trocknen und bei Temperaturen im Bereich von 150 bis 500°C, wobei in Einzelfällen auch Temperaturen von bis zu 1000°C in Betracht kommen können, vorzugsweise 200 bis 450°C in einem Gasstrom aus Luft oder Stickstoff in hierfür geeigneten Apparaturen wie Horden- oder Drehrohröfen calcinieren.

Bei der Herstellung von Tabletten können organische oder anorganische Hilfsmittel wie Stearate, Graphit oder Talkum zugegeben werden.

Nach dem Calcinieren setzt man die Katalysatormasse einer reduzierenden Atmosphäre aus ("Aktivierung"), indem sie beispielsweise bei einer Temperatur im Bereich von 150 bis 300°C, vorzugsweise von 200 bis 280°C 2 bis 96 Stunden einer Wasserstoff-Atmosphäre oder einer Gasmischung, enthaltend Wasserstoff und ein Inertgas wie Stickstoff, aussetzt. Die Katalysatorbelastung beträgt hierbei 200 bis 2000 l pro l Katalysator und pro Stunde.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 150 bis 300, vorzugsweise von 200 bis 280°C in einer Wasserstoff-haltigen Atmosphäre vor.

Die Katalysatoren enthalten
(a) metallisches Cobalt, eine Cobalt-Verbindung oder deren Gemische, wobei der Anteil an metallischem Cobalt, bezogen auf (a) 20 bis 100 Gew.-%, vorzugsweise 30 bis 90 Gew.-%, insbesondere 40 bis 70 Gew.-% beträgt,
(b) 10 bis 70 Gew.-% bezogen auf (a) metallisches Eisen, Eisenoxid eine weitere Eisenverbindung oder deren Gemische, wobei der Anteil an Eisenoxid, bezogen auf (b) 20 bis 100 Gew.-%, vorzugsweise 20 bis 80 Gew.-%, insbesondere 30 bis 70 Gew.-% beträgt und
(c) 0 bis 1 Gew.-%, bezogen auf die Summe aus (a) und (b) eine Verbindung auf der Basis eines Alkalimetalls, Erdalkalimetalls oder Zink.

Die Katalysatoren können als Festbettkatalysatoren in Sumpf- oder Rieselfahrweise oder als Suspensionskatalysatoren eingesetzt werden.

Als Ausgangsstoffe im erfindungsgemäßen Verfahren werden aliphatische alpha,omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Nach dem erfindungsgemäßen Verfahren werden die vorstehend beschriebenen Dinitrile I in Gegenwart eines Lösungsmittels unter Verwendung eines Katalysators partiell zu alpha,omega-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Führt man die Umsetzung in einer Suspension durch, wählt man üblicherweise Temperaturen im Bereich von 20 bis 150°C, vorzugsweise von 30 bis 120°C; den Druck wählt man im allgemeinen im Bereich von 2 bis 30, vorteilhaft von 2 bis 20, vorzugsweise von 3 bis 10, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise beim Einsatz von Adiponitril im Bereich von 50 bis 275, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Hydrierung in Suspension kann man diskontinuierlich oder, bevorzugt kontinuierlich, in der Regel in der Flüssigphase durchführen.

Man kann vorzugsweise die partielle Hydrierung diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 120°C und einen Druck in der Regel im Bereich von 2 bis 30, vorzugsweise von 3 bis 20 MPa wählt. Vorteilhaft kann man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durchführen. In einer bevorzugten Ausführungsform wählt man einen Gehalt an Ammoniak im Bereich von 0,5 bis 10, bevorzugt von 0,5 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/l*h. Auch hier kann man durch Veränderung der Verweilzeit den Umsatz und damit die Selektivität gezielt einstellen.

Nach dem erfindungsgemäßen Verfahren erhält man alpha,omega-Aminonitrile in in guten Selektivitäten und mit nur geringen Mengen an Hexamethylendiamin. Des weiteren weisen die erfindungsgemäß eingesetzten Katalysatoren ein deutlich längere Standzeit auf als vergleichbare Katalysatoren aus dem Stand der Technik. Die alpha,omega-Aminonitrile sind wichtige Ausgangsverbindungen zur Herstellung von cyclischen Lactamen, insbesondere 6-Aminocapronitril für Caprolactam.

### Beispiele

Die Phasenzusammensetzungen der Katalysatoren wurde durch XRD bestimmt.
- Es bedeuten:: ADN = Adipolinitril, HMD = Hexamethylendiamin,
ACN = 6-Aminocapronitril

### Beispiel 1:

Ein Rohrreaktor von 1800 mm Länge und einem Innendurchmesser von 30 mm wurde mit 740 ml (720 g) Katalysator, bestehend aus 48 % CoO, 0,6 % Na₂O, der Rest Fe₂O₃ befüllt. Der Katalysator wurde bei 230°C in einem Wasserstoff/Stickstoffstrom drucklos aktiviert. Anfänglich betrug hierbei der N₂-Strom 450 l/h und der H₂-Strom 50 l/h. Innerhalb der nächsten 8 h wurde der H₂-Anteil am Reduktionsgas kontinuierlich auf 100% erhöht. Nach 8 h bestand der Reduktionsstrom nur noch aus Wasserstoff. Anschließend wurden weitere 12 h bei 250°C mit 500 l/h H₂ drucklos aktiviert.

Nach Absenkung der Temperatur auf 65°C (Eingang) bzw. 80°C (Ausgang) wurde dem Reaktor bei 200 bar ein Gemisch aus 400 ml/h Adipodinitril, 640 ml/h Ammoniak und 500 l/h Wasserstoff in Sumpffahrweise zugeführt. Zur Abfuhr der Reaktionswärme wurden 4 von 5 l Reaktionsaustrag gekühlt und in den Reaktor zurückgefahren. Das Adipodinitril setzte sich unter diesen Bedingungen zu 75 % um. Das Reaktionsgemisch bestand im wesentlichen aus 25 % ADN, 37 % ACN und 37 % HMD. Der Katalysator zeigte auch nach 2600 h bei unveränderter Aktivität noch die gleiche Selektivität wie der Frischkatalysator.

Der Anteil an metallischem Cobalt an Komponente (a) betrug 50 Gew.-%, der Anteil von Eisenoxid an Komponente (b) 30 Gew.-%.

### Vergleichsbeispiel 1:

Drei in Reihe geschaltete Rohrreaktoren (Gesamtlänge 4,5 m d=6 mm) wurden mit 90 ml (107 g) Katalysator aus Beispiel 1 befüllt und anschließend drucklos im Wasserstoffstrom (200 l/h) reduziert. Hierzu wurde die Temperatur innerhalb von 24 h von 50°C auf 340°C angehoben und anschließend 72 h bei 340°C gehalten. Nach Absenken der Temperatur auf 110°C wurde dem Reaktor bei 200 bar ein Gemisch aus 50 ml/h ADN, 280 ml NH₃ und 200 Nl/h H₂ zugeführt. Es konnte kein Umsatz erzielt werden.

Der Anteil an metallischem Cobalt an Komponente (a) betrug 90 Gew.-%, der Anteil von Eisenoxid an Komponente (b) 16 Gew.-%.

### Vergleichsbeispiel 2:

Drei in Reihe geschaltete Rohrreaktoren (Gesamtlänge 4,5 m, d = 6 mm) wurden mit 90 ml (107 g) Katalysator aus Beispiel 1 befüllt und anschließend drucklos im Wasserstoffstrom (200 l/h) reduziert. Hierzu wurde die Temperatur innerhalb von 3 h von 50°C auf 200°C angehoben und anschließend 12 h bei 200°C gehalten. Nach Absenken der Temperatur auf 75°C wurde dem Reaktor bei 200 bar ein Gemisch aus 50 ml/h ADN, 280 ml NH₃/h und 200 Nl/h H₂ zugeführt. Unter diesen Bedingungen wurde ein ADN-Umsatz von 50 % erzielt. Das Reaktionsgemisch bestand im wesentlichen aus 50 % ADN, 40 % ACN und 10 % HMD. Ein solcher Reaktionsaustrag wurde über eine Laufzeit von 300 h erhalten. Nach 300 h wurden die Zuläufe bis auf NH₃ und H₂ gestoppt. Nach einer Spülfahrt von 12 h wurde auch der NH₃-Zulauf gestoppt und der Katalysator auf 340°C und 200 l/h H₂ drucklos über 72 h nachaktiviert. Hierzu wurde die Temperatur innerhalb von 24 h von 50°C auf 340°C angehoben und anschließend 72 h bei 340°C gehalten. Nach Absenken der Temperatur auf 80°C wurde dem Reaktor bei 250 bar ein Gemisch aus 50 ml/h ADN, 230 ml/h NH₃ und 200 ml/h H₂ zugeführt. Es konnte unter diesen Bedingungen und nach Anheben der Temperatur auf 120°C kein Umsatz erzielt werden.

## Patentansprüche

1. Für die Herstellung von aliphatischen alpha, omega-Aminonitrilen durch partielle Hydrierung von aliphatischen Dinitrilen geeignete Katalysatoren, enthaltend
(a) metallisches Cobalt, eine Cobalt-Verbindung oder deren Gemische, wobei der Anteil an metallischem Cobalt bezogen auf (a) 20 bis 100 Gew.-% beträgt,
(b) 10 bis 70 Gew. -% bezogen auf (a) metallisches Eisen, Eisenoxid, eine weitere Eisen-Verbindung oder deren Gemische, wobei der Anteil an Eisenoxid bezogen auf (b) 20 bis 100 Gew. -% beträgt,
(c) 0 bis 1 Gew. -% bezogen auf die Summe aus (a) und (b) eine Verbindung auf der Basis eines Alkalimetalls, Erdalkalimetalls oder Zink.

2. Katalysator nach Anspruch 1, dadurch gekennzeichnet, daß der Katalysator ein Vollkatalysator ist.

3. Katalysator nach Anspruch 1, dadurch gekennzeichnet, daß der Katalysator ein Trägerkatalysator ist.

4. Verfahren zur Herstellung von aliphatischen alpha, omega-Aminonitrilen durch partielle Hydrierung von aliphatischen Dinitrilen bei erhöhter Temperatur und erhöhtem Druck in Gegenwart eines Katalysators gemäß den Ansprüchen 1 bis 3.

5. Verfahren nach Anspruch 4, dadurch gekennzeichnet, daß man die Hydrierung in einem Festbettreaktor vornimmt.

6. Verfahren nach den Ansprüchen 4 oder 5, dadurch gekennzeichnet, daß man als alpha, omega-Dinitril Adipodinitril einsetzt unter Erhalt von 6-Aminocapronitril.

7. Verfahren nach den Ansprüchen 4 bis 6, dadurch gekennzeichnet, daß man die Hydrierung bei einem Druck im Bereich von 2 bis 30 MPa durchführt.

8. Verfahren nach den Ansprüchen 4 bis 7, dadurch gekennzeichnet, daß man die Hydrierung bei einer Temperatur im Bereich von 20 bis 150°C durchführt.

9. Verwendung von Katalysatoren gemäß den Ansprüchen I bis 3 zur Herstellung von aliphatischen alpha, omega-Aminonitrilen durch partielle Hydrierung von aliphatischen Dinitrilen bei erhöhter Temperatur und erhöhtem Druck.

## Claims

1. A catalyst suitable for preparing aliphatic alpha, omega-aminonitriles by partial hydrogenation of aliphatic dinitriles, comprising
(a) metallic cobalt, a cobalt compound or a mixture thereof, the proportion of metallic cobalt based on (a) being from 20 to 100 % by weight,
(b) from 10 to 70 % by weight, based on (a), of metallic iron, iron oxide, a further iron compound or a mixture thereof, the proportion of iron oxide based on (b) being from 20 to 100 % by weight,
(c) from 0 to 1 % by weight, based on the sum of (a) and (b), of a compound based on an alkali metal, an alkaline earth metal or zinc.

2. A catalyst as claimed in claim 1 in the form of an unsupported catalyst.

3. A catalyst as claimed in claim 1 in the form of a supported catalyst.

4. A process for preparing aliphatic alpha, omega-aminonitriles by partial hydrogenation of aliphatic dinitriles at elevated temperature and elevated pressure in the presence of a catalyst as claimed in any of claims 1 to 3.

5. A process as claimed in claim 4, wherein the hydrogenation is effected in a fixed bed reactor.

6. A process as claimed in claim 4 or 5, wherein adiponitrile is used as the alpha, omega-dinitrile to obtain 6-aminocapronitrile.

7. A process as claimed in any of claims 4 to 6, wherein the hydrogenation is carried out at a pressure within the range from 2 to 30 MPa.

8. A process as claimed in any of claims 4 to 7, wherein the hydrogenation is carried out at a temperature within the range from 20 to 150°C.

9. The use of a catalyst as claimed in any of claims 1 to 3 for preparing aliphatic alpha, omega-aminonitriles by partial hydrogenation of aliphatic dinitriles at elevated temperature and elevated pressure.

## Revendications

1. Catalyseurs appropriés pour la préparation d'alpha, oméga-aminonitriles aliphatiques par hydrogénation partielle de dinitriles aliphatiques, contenant
(a) du cobalt métallique, un composé de cobalt ou leurs mélanges, la fraction de cobalt métallique par rapport à (a) étant de 20 à 100% en poids,
(b) 10 à 70% en poids par rapport à (a) de fer métallique, d'oxyde de fer, d'un autre composé du fer ou de leurs mélanges, la fraction d'oxyde de fer par rapport à (b) étant de 20 à 100% en poids,
(c) 0 à 1 % en poids par rapport à la somme de (a) et de (b) d'un composé à base de métal alcalin, de métal alcalino-terreux ou de zinc.

2. Catalyseur suivant la revendication 1, caractérisé en ce que le catalyseur est un catalyseur massique.

3. Catalyseur suivant la revendication 1, caractérisé en ce que le catalyseur est un catalyseur sur support.

4. Procédé de préparation d'alpha, oméga-aminonitriles aliphatiques par hydrogénation partielle de dinitriles aliphatiques à température élevée et pression élevée, en présence d'un catalyseur suivant l'une des revendications 1 à 3.

5. Procédé suivant la revendication 4, caractérisé en ce qu'on effectue l'hydrogénation dans un réacteur à lit fixe.

6. Procédé suivant l'une des revendications 4 et 5, caractérisé en ce que, comme alpha, oméga-dinitriles, on met en oeuvre de l'adipodinitrile avec obtention de 6-aminocapronitriles.

7. Procédé suivant l'une des revendications 4 à 6, caractérisé en ce qu'on effectue l'hydrogénation à une pression de l'ordre de 2 à 30 MPa.

8. Procédé suivant l'une des revendications 4 à 7, caractérisé en ce qu'on effectue l'hydrogénation à une température de l'ordre de 20 à 150°C.

9. Utilisation de catalyseurs suivant l'une des revendications 1 à 3, pour la préparation d'alpha, oméga-aminonitriles aliphatiques par hydrogénation partielle de dinitriles aliphatiques, à température élevée et à pression élevée.
